# EUROPEAN PATENT APPLICATION

(11) **EP 3 998 330 A1**
(43) Date of publication of application: **18.05.2022**
(21) Application number: 20840393.1
(22) Date of filing: 08.07.2020
(51) Int. Cl.: C12M 1/00, C12M 1/34, C12N 1/10, C12N 1/12, C12N 1/14, C12N 1/20

(54) **MICROORGANISM SCREENING SYSTEM AND MICROORGANISM SCREENING METHOD**

(30) Priority: 12.07.2019 JP 2019130223
(71) Applicant: IHI Corporation, Tokyo 135-8710 (JP)
(72) Inventor: IKEDA Ryousuke, Tokyo 135-8710 (JP); ISO Yoshiyuki, Tokyo 135-8710 (JP)
(74) Representative: TBK
(86) International application number: PCT/JP2020/026701
(87) International publication number: WO 2021/010254

(57) **Abstract**

A microorganism screening system includes a container configured to store a liquid containing microorganism particles and a liquid medium and a microorganism separation device. The microorganism separation device includes a hydrodynamic separation device and a liquid feeding unit configured to supply the liquid from the container to the hydrodynamic separation device. The hydrodynamic separation device includes a curved flow channel having a rectangular cross-section, and is configured to separate the liquid into a first segment containing relatively large microorganism particles and a second segment containing relatively small microorganism particles through use of a vortex flow generated in the liquid flowing through the curved flow channel. Screening for microorganisms can be performed efficiently.

## Description

### TECHNICAL FIELD

The present disclosure relates to a microorganism screening system and a microorganism screening method for separating microorganisms contained in a liquid and performing screening for useful microorganism strains in an efficient manner.

### BACKGROUND ART

In recent years, utilization of a useful substance produced by a microorganism has gained attention in various fields including the pharmaceutical industry. In order to achieve availability of such a useful substance in the market, various devices and improvements have been made to suitable and efficient conditions for microorganism culture, an isolation/refinement technique for a produced useful substance, and the like.

Examples of microorganisms used in the industry include bacteria such as a colon bacillus and fungi such as yeast, and studies with regard to screening for selecting useful microorganisms from various microorganisms have been developed. When microorganisms are utilized, it is effective to perform screening and obtain a particular strain with high a proliferation function, productivity, and the like. In the prior art, screening for microorganism strains is generally performed through picking with visual inspection. Each of picked strains is cultured, and a strain to be utilized is determined based on a result of a performance test conducted for each strain (see Non-patent Literatures 1 to 3 given below). This screening operation requires a technique of a skilled person, and also requires labor and time.

As a literature with regard to separation of particles contained in a fluid, Patent Literature 1 is given below that describes a hydrodynamic separation device including a curved channel. In this device, a fluid containing particles is supplied to the curved channel, and the particles can be separated through use of a force acting on the fluid flowing through the curved channel.

### CITATION LIST

### PATENT LITERATURE

Patent Literature 1: Japanese Unexamined Patent Application Publication (Translation of PCT Application) No. 2016-526479

### NON-PATENT LITERATURE

Non-patent Literature 1: Miyashita Hideaki, Araya Shogo, Imura Ayako, Yuan Chen, Ishii Kenichiro & Kamikawa Ryoma. Isolation and Selection of Microalgae Suitable for an Attached Culture System, a Possible Next Generation Biomass/biodiesel Production System. Nihon Enerugii Gakkai kikanshi Enermix, Vol. 96, 40-49 (2017).
Non-patent Literature 2: Ebina Sayaka, Yamazaki Harutake, Shida Yosuke, Ogasawara Wataru, & Takaku Hiroaki. O-20 Isolation of Industrial Oleaginous yeast Lipomyces starkeyi Mutants Accumulating a High Level of Lipid. Baiomasu Kagaku Kaigi happyo ronbunshu, The Eleventh Baiomasu Kagaku Kaigi O-20, (2016)
Non-patent Literature 3: Naganuma, Takafumi. & Yanagiba, Mana, Prospect of Practical Productivity of Oil and Fat for BDF through Utilization of Lipomyces Yeast subjected to Screening and Renewable Resource. Seibutu-kougaku Kaishi, Vol. 94, 324-328, (2016).

### SUMMARY OF THE INVENTION

As described above, for industrial utilization of microorganisms, it is important to perform screening for microorganisms in an efficient manner. The separation technique described in Patent Literature 1 relates to separation of solid particles. In this regard, its availability for microorganisms is unknown.

Strains obtained through screening are used to confirm a proliferation function and productivity thereof. Thus, when microorganisms are subjected to screening to obtain active strains, it is required to avoid damages on the microorganisms. In this regard, when the separation technique is applied to microorganisms, a sufficient examination on its impact is required.

It is an object of the present disclosure to provide a microorganism screening system and a microorganism screening method that enable efficient screening for microorganisms, allow microorganisms after screening to suitably maintain a proliferation function, and enable confirmation of a property thereof.

### SOLUTION TO PROBLEM

In order to achieve the above-mentioned object, there has been examined an impact on microorganisms, which is caused at the time of performing separation of particle-like microorganisms dispersed in a liquid. As a result, it has been found that screening for microorganism strains and a performance test can be achieved by separating microorganism particles efficiently in accordance with a particle size through use of a hydrodynamic separation technique.

According to one aspect of the present disclosure, a microorganism screening system includes a container configured to store a liquid containing microorganism particles and a liquid medium and a microorganism separation device. The microorganism separation device includes a hydrodynamic separation device and a liquid feeding unit. The hydrodynamic separation device includes a curved flow channel having a rectangular cross-section, and is configured to separate the liquid into a first segment containing relatively large microorganism particles and a second segment containing relatively small microorganism particles through use of a vortex flow generated in the liquid flowing through the curved flow channel. The liquid feeding unit is configured to supply the liquid from the container to the hydrodynamic separation device.

The hydrodynamic separation device may be configured to include a single inlet port through which the liquid is takes in and two outlet ports through which the first segment and the second segment are separately discharged. The relatively large microorganism particles are concentrated and contained in the first segment. The liquid feeding unit may include a pipe configured to connect the container and the hydrodynamic separation device to each other and a pump configured to apply a hydrodynamic pressure to the liquid for supplying the liquid to the hydrodynamic separation device.

The microorganism screening system may further include a test device configured to measure a size of a microorganism particle contained in a liquid. A flow rate of the liquid supplied from the liquid feeding unit to the hydrodynamic separation device is changed based on a measurement result on at least one of the first segment and the second segment, the measurement result being obtained by the test device. The liquid feeding unit may include a flow rate control mechanism configured to control a flow rate of the liquid supplied to the hydrodynamic separation device. It is suitable that the test device further includes means for measuring at least one of the number of particles, particle diameter distribution, and a survival rate of microorganism particles.

The microorganism screening system may further include at least one additional hydrodynamic separation device including a curved flow channel with a curvature or a rectangular cross-sectional shape being different from that of the hydrodynamic separation device. The liquid feeding unit may include a supply switching mechanism configured to supply the liquid to one of the hydrodynamic separation device and the additional hydrodynamic separation device and, at the same time, change a supply destination. Further, the microorganism screening system may further include a test device configured to measure a size of a microorganism particle contained in a liquid. The supply switching mechanism is configured to change a supply destination of the liquid based on a measurement result on at least one of a first segment and a second segment that are obtained by separating a liquid supplied to one of the hydrodynamic separation device and the additional hydrodynamic separation device, the measurement result being obtained by the test device.

Moreover, according to one aspect of the present disclosure, a microorganism screening method is a microorganism screening method of performing screening for microorganism particles in accordance with a particle size through use of a liquid containing the microorganism particles and a liquid medium. The microorganism screening method includes a hydrodynamic separation step of supplying the liquid to a curved flow channel having a rectangular cross-section and separating the liquid into a first segment containing relatively large microorganism particles and a second segment containing relatively small microorganism particles through use of a vortex flow generated in the liquid flowing through the curved flow channel and an isolation step of isolating at least one of the first segment and the second segment.

The microorganism particles described above may contain any one of a virus, a eubacterium, an archaebacterium, a fungus, a myxomycete, an alga, and a protist. Further, the microorganism particles are a single species, and screening for target microorganism strains is performed through separation in the hydrodynamic separation device.

### ADVANTAGEOUS EFFECTS

According to the present disclosure, particle-like microorganisms dispersed in a liquid can be separated efficiently. Simultaneously, through use of the microorganisms after separation, a test for the microorganism strains can be conducted efficiently. Further, microorganisms can be separated in a sequential and aseptic manner, and hence contamination can be prevented during screening for microorganisms. Therefore, the microorganism screening system and the microorganism screening method that enable efficient screening for microorganism strains are provided, and hence useful substances can be obtained efficiently through selective utilization of superior microorganism strains.

### BRIEF DESCRIPTION OF DRAWINGS

[FIG. 1] FIG. 1 is a schematic configuration diagram showing a microorganism screening system according to a first embodiment.
[FIG. 2] FIG. 2 is a schematic configuration diagram showing a microorganism screening system according to a second embodiment.
[FIG. 3] FIG. 3 is a schematic configuration diagram showing a microorganism screening system according to a third embodiment.
[FIG. 4] FIG. 4 is a schematic configuration diagram showing a microorganism screening system according to a fourth embodiment.
[FIG. 5] FIG. 5 is a schematic configuration diagram showing a microorganism screening system according to a fifth embodiment.
[FIG. 6] FIG. 6 is a schematic configuration diagram showing a microorganism screening system according to a sixth embodiment.
[FIG. 7] FIG. 7 is a graph showing a relationship between a De number and separation efficiency in cell separation performed by a hydrodynamic separation device.
[FIG. 8] FIG. 8 is a graph showing a relationship between a pump used for cell separation and a cell survival rate.

### DESCRIPTION OF EMBODIMENTS

Embodiments of the present disclosure are described below in detail with reference to the drawings. Note that dimensions, materials, and other specific numerals in the embodiments are given for easy understanding of the contents, and do not limit the present disclosure unless otherwise noted. Further, in Description and the drawings of the present application, elements having substantially the same function and configuration are denoted with the same reference symbol, and redundant description therefor is omitted. Elements that are not directly related to the present disclosure are omitted in illustration. In addition, in the drawings, connection indicated with the broken line indicates electrical or electronic connection that enables transmission of a control signal or the like in a wired or wireless manner.

Screening for microorganisms is performed to obtain microorganism strains excellent in a proliferation function, a metabolizing function, productivity, or the like. In some methods, a size (cell diameter) is used as a selection parameter. An active microorganism having a high proliferation function tends to be relatively large. Non-patent Literature 3 given above relates to a yeast having productivity of oil and fat, and describes that a yeast having a larger fat globule volume produces a larger amount of oil and fat. Therefore, separation of microorganism particles in accordance with a size is a useful screening method for microorganism strains.

One of the separation techniques for separating particles contained in a liquid uses an effect of a Dean vortex generated in the liquid (see Patent Literature 1 given above; hereinafter, the technique is referred to as hydrodynamic separation). This separation technique utilizes uneven distribution of the particles in the liquid in which a Dean vortex is generated. The liquid flows through a curved flow channel that has a rectangular cross-section vertical to a flow direction and is curved to one side. The particles flowing through the curved flow channel are distributed differently in the flow channel in accordance with a size (see Patent Literature 1 given above). Specifically, ring-like particle distribution is formed in the cross-section of the flow channel, and the particles spirally flow through the flow channel. In this state, a particle having a relatively large size is positioned on the outer side of the ring, and a particle having a relatively small size is positioned on the inner side of the ring. Further, when a predetermined separation condition is set, a particle distribution mode further changes. As a result, particles having a size exceeding a certain level converge on the outer circumferential side of the flow channel.

In the present disclosure, the above-mentioned hydrodynamic separation is applied to separation of microorganism particles, in other words, microorganisms having a particle-like shape contained in the liquid, and is used in a separation mode in which microorganism particles having a predetermined size or larger converge on the outer circumferential side of the curved flow channel. In the microorganism screening system according to the present disclosure, the liquid is supplied to the curved flow channel at a relatively high flow speed. As described above, while the liquid to be supplied flows through the curved flow channel, relatively small microorganism particles are distributed in a ring shape in the cross-section of the flow channel. Meanwhile, relatively large microorganism particles converge evenly on the outer side (outer circumferential side) of the curved flow channel. Therefore, with separation into a first segment in which the larger microorganism particles are concentrated and a second segment being the rest, the liquid in which the relatively large microorganism particles are concentrated can be isolated. The first segment in which the larger microorganism particles are concentrated contains a small amount of the relatively small microorganism particles. However, the concentration of the smaller microorganism particles is significantly reduced from that before the separation. The second segment being the rest contains the rest (most part) of the small microorganism particles. When the liquid is a culture solution for microorganisms, a large amount of metabolites, fine condensates of effete matters, dead cell pieces (debris), and the like that are generated through culturing microorganisms are further contained. As described above, a separation state is adjusted based on a condition for allowing the liquid to flow through, thereby performing separation into relatively large particles and relatively small particles. A size of the concentrated microorganism particles can be adjusted based on settings of a flow speed (flow rate) of the liquid to be supplied and a size of the cross-section of the curved flow channel.

A microorganism grows large in a highly active state. However, an active level is lowered, a microorganism is dead and dissolved while having a relatively small size. Most of the relatively small particles are dead microorganisms or dead microorganism pieces, and the chances that active microorganisms in the course of DNA synthesis are contained are low. Therefore, when the first segment in which the relatively large microorganism particles are concentrated is isolated from the liquid, unnecessary matters such as metabolites are removed, and an amount thereof is reduced. A size of the particles to be separated through hydrodynamic separation can be adjusted by changing a separation condition (a supply flow rate of the liquid). Further, by performing multistep hydrodynamic separation in which the segment containing the relatively large microorganism particles is used to repeat hydrodynamic separation while changing the separation condition, microorganism particles having different sizes can be isolated at the respective steps. Further, by repeating hydrodynamic separation under the same separation condition, separation accuracy can be improved. When the liquid contains unnecessary particles larger than target microorganism particles, separation is performed once under such a separation condition that the unnecessary particles are concentrated in the first segment and the target microorganism particles are contained in the second segment. In this manner, the unnecessary particles can be removed as the first segment. The target microorganism particles are isolated as the second segment, the separation condition is changed, and then hydrodynamic separation is repeated. With this, the microorganisms can be separated in accordance with a size.

In hydrodynamic separation, a flow in the flow channel is a laminar flow. Relatively, fracture of the particles contained in the liquid is less likely to happen, which is highly effective for separation of microorganisms. Still, presence or absence of damage on the microorganisms during separation is a key point to be examined for efficient screening for microorganisms and a sequential performance test. As a result of examination on this point, microorganisms such as a yeast and fungi have resistance under a relatively high pressure, and a survival rate thereof is maintained even when a pressure of, for example, approximately 1 MPa is applied. Moreover, the microorganisms have considerably high durability against pressure fluctuation. In a case where animal cells are cultured, the animal cells are easily affected by fluctuation of a pressure (pressure reduction) applied to the cell during separation, and a survival rate of the cells is lowered. Nevertheless, even when the microorganisms are under considerably large pressure fluctuation during hydrodynamic separation, a survival rate thereof can be maintained. Therefore, during hydrodynamic separation for microorganism particles, it is not particularly required to control a pressure environment. Thus, a hydrodynamic pressure can be changed by changing a driving force of a pump or the like that is used to supply a liquid to a hydrodynamic separation device, and hence a flow rate of the liquid can be adjusted based on a hydrodynamic pressure in a freely selective manner. In other words, settings for the separation condition can be changed easily through drive control of a pump, and hence the system configuration can be easily simplified. Further, hydrodynamic separation has advantages in that an aseptic state of the liquid can be easily maintained and that separation with high reproducibility can be achieved regardless of the skill level of an operator.

In a case where an impact of a pressure environment is concerned while dealing with mutant strains or special microorganisms, fluctuation of a pressure applied to the microorganisms may be controlled during hydrodynamic separation. In this case, the separation condition is set so that fluctuation of a pressure (pressure reduction) applied to the cell during separation does not exceed a certain level. With this, a pressure environment in which a liquid culture medium is supplied to the curved flow channel is controlled. In other words, supply of the liquid is controlled so that fluctuation of a pressure (a difference between an inlet pressure and an outlet pressure) applied to the microorganisms during separation is equal to or lower than a predetermined value. Specifically, control is performed appropriately when pressure fluctuation (pressure difference) is set to be less than 0.60 MPa, preferably, equal to or less than 0.45 MPa, more preferably, equal to or less than 0.40 MPa. Under the separation condition for controlling pressure fluctuation as described above, microorganisms are less damaged, and a survival rate of the microorganisms can be maintained at approximately 98% or higher. Therefore, large microorganism particles after concentration and separation can be isolated, and a proliferation function or the like thereof can be maintained. Under this state, the large microorganism particles can be supplied for measurement of particle diameter distribution, microorganism culture, testing, and the like.

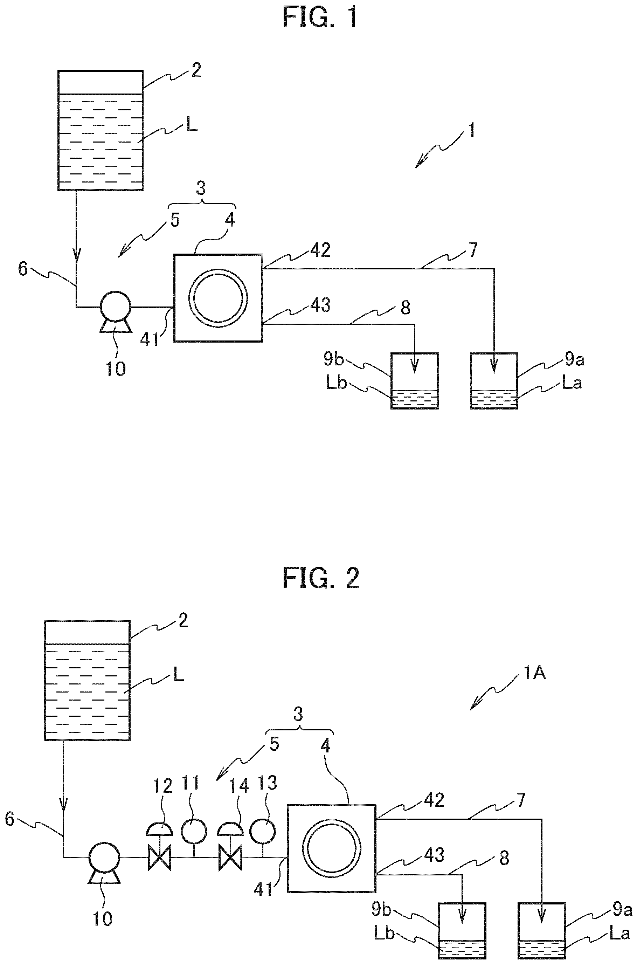

A configuration of the microorganism screening system is described below with reference to the embodiments illustrated in the drawings. A microorganism screening system 1 in FIG. 1 includes a container 2 that stores a liquid containing microorganism particles and a liquid medium and a microorganism separation device 3. The microorganism separation device 3 includes a hydrodynamic separation device 4 and a liquid feeding unit 5. The liquid L in the container 2 is supplied to the hydrodynamic separation device 4 via the liquid feeding unit 5. The hydrodynamic separation device 4 includes a curved flow channel inside, which has a constant rectangular cross-section vertical to a flow direction, and includes a single inlet port 41 at one end of the curved flow channel and at least two outlet ports 42 and 43 at the other end of the curved flow channel. The liquid L is taken in through the inlet port 41, and a liquid culture medium is separated and discharged separately through the outlet ports 42 and 43. While the liquid flows through the curved flow channel, a vortex flow is generated in the liquid swirling in one direction. The hydrodynamic separation device 4 utilizes the vortex flow, and causes relatively large particles among the microorganism particles contained in the liquid L to be evenly present on the outer side (outer circumferential side) of the flow channel. Therefore, the liquid discharged from the curved flow channel can be separated into a first segment on the outer side and a second segment on the inner side. Through separation into the outer segment and the inner segment, a liquid culture medium in which the relatively large microorganism particles are concentrated can be isolated as the outer segment. The liquid culture medium (first segment) in which the relatively large microorganism particles are concentrated and are contained is discharged through the one outlet port 42. A liquid culture medium (second segment) being the rest in which the relatively small microorganism particles are concentrated and are contained is discharged through the other outlet port 43.

The curved shape of the curved flow channel may be a substantially circular shape, a substantially arc shape (partial circumference), a spiral shape, or the like, and may be selected freely from those shapes. The hydrodynamic separation device 4 may be designed with a flow channel unit including one curved flow channel as a flow channel unit. Specifically, the flow channel unit is configured in the following manner. A molded body having a plane layer is formed of plastic or the like, and one curved flow channel is formed inside the molded body. Then, the end surfaces of the molded body are opened at both the terminal ends of the curved flow channel, and hence one inlet port and at least two outlet ports are formed. The molded body as described above is used as the flow channel unit. The hydrodynamic separation device may consist of one flow channel unit or a combination of a plurality of flow channel units. When the plurality of flow channel units are layered to form the flow channel in a parallel state, a processing flow rate of the liquid L can be increased.

The liquid feeding unit 5 includes a supply channel 6 formed of a pipe connecting the container 2 and the hydrodynamic separation device 4 to each other. The liquid L containing the microorganism particles in the container 2 is fed to the hydrodynamic separation device 4 through the supply channel 6.

The first segment (liquid La) that contains the relatively large microorganism particles after separation in the hydrodynamic separation device 4 passes through a flow channel 7 from the outlet port 42, and is stored in a recovery container 9a. The liquid Lb (the second segment on the inner circumferential side) being the rest that contains the relatively small microorganism particles is discharged from the outlet port 43 of the hydrodynamic separation device 4 through a flow channel 8, and is stored in a recovery container 9b.

Efficiency in separating the microorganism particles in the hydrodynamic separation device 4 is changed in accordance with a Dean number (De number) and a pressure of the liquid supplied to the curved flow channel. When the De number and the pressure fall within proper ranges, separation can be performed suitably. The De number is expressed in Equation of De=Re(D/2Rc)^{1/2} (Re denotes a Reynold's number (-), D denotes a representative length (m), and Rc denotes a turning radius of the flow channel (m)). The De number is proportional to a flow speed of the liquid. Thus, when a flow speed of the liquid supplied to the hydrodynamic separation device is controlled properly, the microorganisms can be separated suitably. In general, the De number is preferably 30 or greater and 100 or less, more preferably, approximately from 50 to 80. Therefore, a flow speed (flow rate) of the liquid is set so that the De number falls within this range.

The liquid feeding unit 5 of the microorganism screening system 1 includes a pressurizing device that applies a hydrodynamic pressure to the liquid L, specifically, a pump 10. With the hydrodynamic pressure, the liquid Lis supplied to the hydrodynamic separation device 4. The hydrodynamic pressure applied by the pump 10 changes a flow rate and a flow pressure at which the liquid L is supplied to the hydrodynamic separation device 4. A survival rate of the microorganisms is an important factor when screening for microorganism strains is performed through use of the microorganism particles after separation. In this regard, many of the microorganisms have durability against separation performed by the hydrodynamic separation device 4, and a survival rate can be maintained. However, in a case where it is desired that an impact of pressure fluctuation be eliminated, a microorganism screening system 1A is configured as in FIG. 2, for example. With this, a pressure environment can be controlled.

In FIG. 2, the liquid feeding unit 5 includes a pressure control mechanism. With this, a pressure environment is controlled so that a pressure difference between the liquid introduced into the hydrodynamic separation device 4 and the liquid exiting from the hydrodynamic separation device 4 is equal to or less than a predetermined value. The pressure control mechanism may include a pressure monitoring unit and a pressure adjusting member. The pressure monitoring unit monitors a pressure of the liquid supplied to the hydrodynamic separation device 4, and the pressure adjusting member adjusts a pressure of the liquid supplied to the hydrodynamic separation device 4 based on the pressure monitored by the pressure monitoring unit. Specifically, in the microorganism screening system 1A, a pressure gauge 11 and a pressure adjusting valve 12 are provided in the supply channel 6 as the pressure monitoring unit and the pressure adjusting member, respectively. In the microorganism screening system 1A, an outlet-side pressure at the outlet ports 42 and 43 of the hydrodynamic separation device 4 is opened to an atmospheric pressure. Thus, a pressure difference between the introduced liquid and the exiting liquid is equal to a pressure (gauge pressure) measured by the pressure gauge 11. Therefore, pressure control can be performed based on a measurement value of a pressure through use of the pressure adjusting valve 12. When a pressure environment is controlled so that a pressure difference is less than 0.60 MPa, preferably, equal to or less than 0.45 MPa, more preferably, equal to or less than 0.40 MPa, reduction of a survival rate and possible damage of the microorganisms during separation can be prevented.

As described above, separation efficiency in the hydrodynamic separation device 4 depends on a flow speed of the liquid flowing through the curved flow channel. Therefore, the liquid feeding unit 5 further includes a flow rate control mechanism that controls a flow rate of the liquid supplied to the hydrodynamic separation device 4. With this, a flow rate of the liquid flowing through the supply channel 6 is controlled so that the liquid flowing through the curved flow channel of the hydrodynamic separation device 4 has a proper flow speed. The flow rate control mechanism includes a flowmeter 13 and a flow rate adjusting valve 14. The flowmeter 13 monitors a flow rate of the liquid L supplied to the hydrodynamic separation device 4. The flow rate adjusting valve 14 functions a flow rate adjusting member that adjusts, based on the flow rate monitored by the flowmeter 13, the flow rate of the liquid supplied to the hydrodynamic separation device 4. In accordance with a size of the microorganism particles subjected to separation, the flow rate of the liquid supplied to the hydrodynamic separation device 4 is adjusted. This adjustment can be performed under a state in which a constant drive of the pump 10 is maintained.

In the microorganism screening system 1 in FIG. 1, drive control of the pump 10 is utilized in place of the flow rate adjusting valve so that the flow rate of the liquid supplied to the hydrodynamic separation device 4 is adjusted properly. Further, based on durability of the microorganisms against a pressure environment, the pressure control mechanism is omitted. A supply flow rate and a pressure of the liquid can be regulated to some extent by designing dimensions of the supply channel 6 and the flow channels 7 and 8 properly based on processing capacity (a size of the cross-section of the flow channel and the number of flow channels) of the hydrodynamic separation device 4. In this manner, even with a simple configuration as in the microorganism screening system 1 in FIG. 1, the microorganism particles can be separated suitably, and efficient and sequential screening for microorganism strains can be performed.

Microorganisms have resistance against a high static pressure to some extent. As illustrated in FIG. 1, in a case where the flow rate of the liquid supplied to the hydrodynamic separation device 4 is adjusted through drive control of the pump 10, when the flow rate is increased, an inlet pressure of the liquid is also increased. When it is required to set the inlet pressure to a high value without escalating pressure fluctuation during separation, a pressure adjusting valve may also be provided on the outlet side of the hydrodynamic separation device 4, that is, the flow channels 7 and 8. With this, the outlet pressure may be adjusted. The outlet pressure of the liquid discharged from the outlet ports 42 and 43 of the hydrodynamic separation device 4 can be increased, and hence a pressure difference between the inlet pressure and the outlet pressure can be reduced. In this case, it is suitable to provide a pressure gauge to each of the flow channel 7 and the flow channel 8 and monitor a discharge pressure so as to obtain an appropriate pressure difference. In this state, for the liquid flowing through the flow channel 7 and the flow channel 8 downstream of the pressure adjusting valves, a pressure environment may also be checked so as not to cause sudden pressure fluctuation.

The container 2 and the recovery containers 9a and 9b are containers capable of preventing microorganism contamination. For each of the containers, a container equipped with a heater or a cooler and having a temperature adjusting function may be used as required. The liquid stored inside is maintained at a temperature suitable for culturing or storing microorganisms. The container 2 may include a stirring device for uniformizing the liquid. With this, stirring can be performed at such an appropriate speed that the microorganisms are not damaged. Alternatively, in order to maintain an environment suitable for the microorganisms, a container having a function of adjusting an amount of oxygen/carbon dioxide/air, pH, conductivity, a light amount, or the like may be used as required.

In the microorganism screening system, when an impact on the microorganisms is eliminated as much as possible, a pump of such a type that does not apply a shearing force to the microorganisms may be used. Specifically, it is suitable to use a positive displacement pump that utilizes a volume change caused by reciprocating motion or rotating motion and push out a liquid having a predetermined volume. Examples of the positive displacement pump include a reciprocating pump such as a piston pump, a plunger pump, a diaphragm pump, and a wing pump, and a rotary pump such as a gear pump, a vane pump, and a screw pump. In one embodiment, for example, a pressure tank to which a compressor is attached may be used. In this embodiment, a liquid stored in the pressure tank can be pressurized by the compressor, and then the liquid can be forcibly fed from the pressure tank to the hydrodynamic separation device.

The liquid stored in the container 2 is a liquid containing microorganism particles and a liquid medium. The microorganism particles are microorganisms having a particle-like shape. Specifically, the microorganism particles may be freely selected particles single cells or a plurality of cells of microorganisms. The microorganisms are separated in accordance with a particle size during hydrodynamic separation. The liquid medium may be any liquid as long as the microorganisms are not damaged, and may contain any component harmless to the microorganisms. In general, an aqueous liquid such as water, fresh water, and sea water is suitable, and a liquid culture medium used for microorganism culture may be used. Therefore, the microorganisms cultured in the liquid culture medium may be directly introduced into the microorganism screening system.

As described above, separation of microorganism particles in accordance with a size is a useful screening method for microorganism strains. The first segment in which the relatively large microorganism particles are concentrated through hydrodynamic separation is isolated based on this method. Then, the segment may contain microorganism strains excellent in a proliferation function, a metabolizing function, productivity, or the like. Further, the obtained first segment is repeatedly subjected to hydrodynamic separation, thereby improving particle separation accuracy. Further, when unnecessary particles larger than the target microorganisms are contained in the liquid, the unnecessary particles are removed as the first segment, and the second segment is repeatedly subj ected to hydrodynamic separation. With this, the target microorganism particles can be isolated.

A microorganism screening method that can be performed in the microorganism screening system 1 described above is described below. The microorganism screening method is a method of performing screening for microorganisms. In the method, a liquid containing microorganism particles and a liquid medium is used, and screening for microorganisms is performed in accordance with a particle size. In other words, the microorganism screening method includes a hydrodynamic separation step of separating the liquid into the first segment containing the relatively large microorganism particles and the second segment containing the relatively small microorganism particles and an isolation step of isolating at least one of the first segment and the second segment. The hydrodynamic separation step takes place in the hydrodynamic separation device 4. The isolation step takes place in the flow channels 7 and 8 and the recovery containers 9a and 9b. In the hydrodynamic separation step, the liquid is supplied to the curved flow channel having a rectangular cross-section, and the microorganism particles are separated in accordance with a particle size through use of a vortex flow generated in the liquid flowing through the curved flow channel.

In the hydrodynamic separation step, the liquid containing the microorganism particles is introduced through a single inlet port into the curved flow channel having a rectangular cross-section, and is supplied to the curved flow channel under a uniformed state. The curved flow channel of the hydrodynamic separation device is a flow channel having a rectangular cross-section vertical to the flow direction (radial cross-section). While the uniform liquid flows through the curved flow channel, the relatively small particles flow with a Dean vortex, and distribution thereof is changed to a ring-like shape in the rectangular cross-section. Meanwhile, the relatively large particles are distributed to the outer circumferential side in a concentrated manner because a dynamic lifting force stagnating on the outer circumferential side of the flow channel acts thereon in a relatively intense manner. The terminal end outlet of the curved flow channel is divided into two ports, namely, the outlet port 42 positioned on the outer circumferential side and the outlet port 43 positioned on the inner circumferential side. The liquid in which the relatively large microorganism particles are concentrated and are contained is discharged through the outlet port 42 on the outer circumferential side. The liquid culture medium being the rest in which the relatively small microorganism particles are contained is discharged through the outlet port 43 on the inner circumferential side.

In the isolation step, the first segment is isolated. With this, the relatively large microorganism particles, in other words, the microorganism strains excellent in a proliferation function, a metabolizing function, productivity, or the like can be obtained. When the liquid subjected to screening contains particles larger than the target microorganism particles, unnecessary particles can be removed as the first segment, and the target microorganism particles can be obtained as the second segment.

As shown in Expression given above, the De number is changed in accordance with the turning radius Rc of the curved flow channel and the dimension of the cross-section of the flow channel (the representative length D in Expression given above can be regarded as the width of the curved flow channel). Therefore, adjustment can be performed based on the design of the curved flow channel so as to obtain a suitable value for the De number. With this, the hydrodynamic separation device can perform concentration and separation of cells with satisfactory separation efficiency. Further, the flow rate of the liquid can be adjusted in accordance with the settings of any one of the width and the height of the cross-section (radial cross-section) of the curved flow channel. Thus, the hydrodynamic separation device may be configured based on the design of the curved flow channel so that separation of the microorganism particles can be performed at a desired flow rate. Therefore, the design of the curved flow channel may be changed as appropriate so that suitable separation is achieved in accordance with conditions of the separation target (dimension distribution of microorganisms, viscosity of a liquid culture medium, and the like). From a viewpoint of efficiency in separating the microorganism particles, it is suitable that the curved flow channel is a flow channel with a rectangular cross-section having an aspect ratio (width/height) of 10 or greater. The liquid is supplied to the curved flow channel described above at a flow rate of approximately 100 mL to 500 mL per minute. With this, separation progresses satisfactorily, and the microorganism particles can be subjected to separation processing at efficiency of approximately five billion to twenty-five billion cells per minute. Microorganism particles having a particle diameter of approximately 10 µm or larger can be concentrated and isolated as the relatively large microorganism particles in the segment on the outer circumferential side. Most of the relatively small microorganism particles that are distributed in a ring shape and have a size of approximately 5 µm or smaller can be isolated as the segment on the inner circumferential side. With the design of the terminal end outlet of the curved flow channel (positions of the divided outlet ports), a size and separation accuracy of the microorganism particles contained in the segment on the outer circumferential side can be adjusted. Similarly, the lower limit size of the microorganism particles contained in the segment on the outer circumferential side can be smaller than 10 µm. Larger microorganism cells have a higher survival rate and are more active than smaller cells. When the segment on the outer circumferential side is isolated, an amount of less active cells, dead cells, and cell pieces can be reduced. Therefore, when the liquid containing the relatively large microorganism particles after separation in the hydrodynamic separation step is isolated, screening for desired microorganism strains can be performed. The hydrodynamic separation step is repeated as required, thereby improving particle separation accuracy.

A separation state of the microorganism particles in the hydrodynamic separation device (a size and an isolation ratio of the microorganism particles to be isolated) differs depending on positions of the divided outlet ports. In general, a cross-sectional area ratio of the flow channel outlet is designed so that a division ratio (volume ratio) of the segment on the outer circumferential side/the segment on the inner is approximately 10/90 to 70/30, which is suitable for concentration and separation for the microorganism particles as described above. In the microorganism screening system in FIG. 1 and FIG. 2, the hydrodynamic separation device has the two outlet ports as the outlet of the curved flow channel, but may have three or more divided outlet ports. When the terminal end outlet is divided into three or more outlet ports, a configuration in which an amount of the isolated segment can be changed in accordance with a circumstance may be adopted.

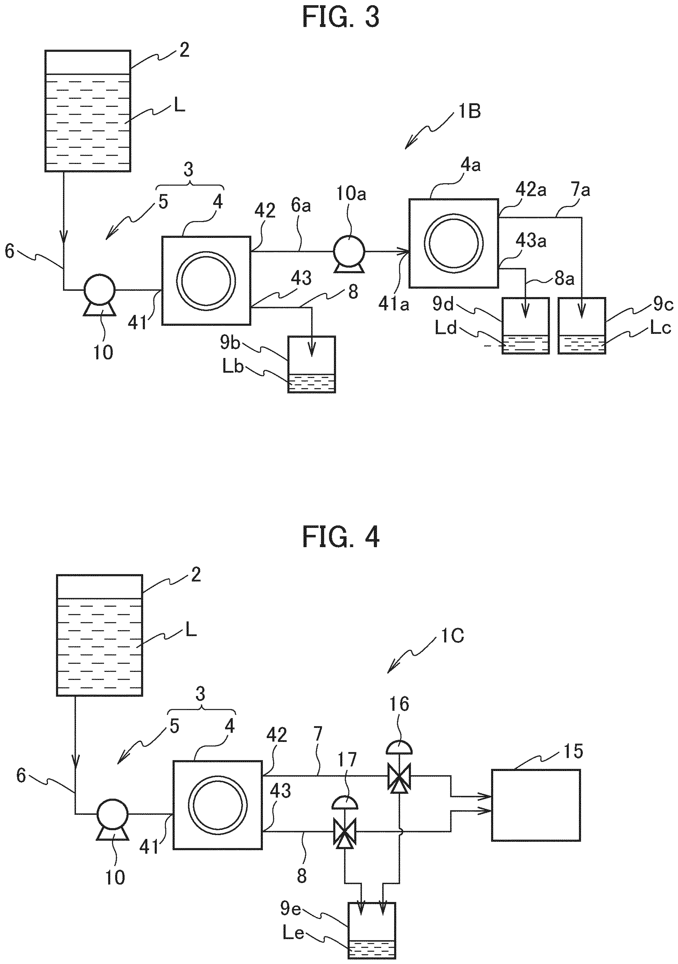

A microorganism screening system 1B in FIG. 3 is configured to perform two-step separation processing through use of two hydrodynamic separation devices 4 and 4a. Specifically, the hydrodynamic separation device 4a for the second step is further provided. With this, the liquid La in the first segment discharged from the hydrodynamic separation device 4 after separation of the liquid L, that is, the segment containing the relatively large microorganism particles on the outer circumferential side is further subjected to separation. The outlet port 42 of the hydrodynamic separation device 4 for the first step is connected to an inlet port 41a of the hydrodynamic separation device 4a for the second step through intermediation of a supply channel 6a. The supply channel 6a is provided with a pump 10a. The liquid La (first segment) is discharged from the outlet port 42, and is supplied to the inlet port 41a of the hydrodynamic separation device 4a by the pump 10a. Then, the liquid La is further divided into two segments. A segment containing relatively large particles (on the outer circumferential side) among the microorganism particles contained in the liquid La passes through a flow channel 7a from an outlet port 42a, and is stored in a recovery container 9c. A liquid Ld being the rest that contains relatively small particles (the segment on the inner circumferential side) passes through a flow channel 8a from an outlet port 43a, and is supplied to a recovery container 9d.

In the microorganism screening system 1B in FIG. 3, the two-step separation is performed, and the components contained in the liquid is divided into three groups. The separation condition in the hydrodynamic separation device 4a for the second step can be adjusted by controlling a supply flow rate of the liquid La supplied through the supply channel 6a. The separation conditions in the two hydrodynamic separation devices 4 and 4a are set appropriately. With this, separation accuracy and a concentration degree of the microorganism particles can be improved. Note that, in accordance with a division ratio of the two segments in the hydrodynamic separation device for the first step, processing capacity of the hydrodynamic separation device for the second step may be set appropriately.

Note that, when a change is made to the microorganism screening system in FIG. 3 so that the outlet port 43 of the hydrodynamic separation device 4 for the first step is connected to the hydrodynamic separation device 4a for the second step, the second segment can be further subjected to hydrodynamic separation. Therefore, the system in this case may be applied to a liquid containing particles larger than the target microorganism particles.

A microorganism screening system 1C in FIG. 4 corresponds to an embodiment that further includes a test device 15 capable of measuring a size of microorganism particles contained in a liquid. The test device 15 conducts a test for at least one of the first segment and the second segment after separation in the hydrodynamic separation device 4, and thus a particle diameter, particle diameter distribution, and the like are obtained. The flow rate of the liquid L supplied from the liquid feeding unit 5 to the hydrodynamic separation device 4 can be adjusted based on the test results obtained by the test device 15. In other words, the separation condition in the hydrodynamic separation device 4 can be changed and optimized.

Specifically, in the microorganism screening system 1C, each of the flow channel 7 and the flow channel 8, which are connected to the outlet port 42 and the outlet port 43, respectively, is branched to have two terminal ends. Each of the two terminal ends is connected to the test device 15 and a recovery container 9e. A switching valve 16 and a switching valve 17 are provided at the branching points of the flow channel 7 and the flow channel 8, respectively. Thus, with the switching valves 16 and 17, any one of the first segment and the second segment can be supplied to any one of the test device 15 and the recovery container 9e. In other words, the segment subjected to testing by the test device 15 can be selected and changed through switching. The segment that is not supplied to the test device 15 is stored in the recovery container 9e as a liquid Le. Therefore, when both the segments are subjected to testing, switching is performed by the switching valve, and the two segments are sequentially supplied to the test device. Particle diameter measurement by the test device may be performed manually. As illustrated in FIG. 4, the test device is connected through intermediation of a pipe, and hence microorganism contamination before the test can be prevented.

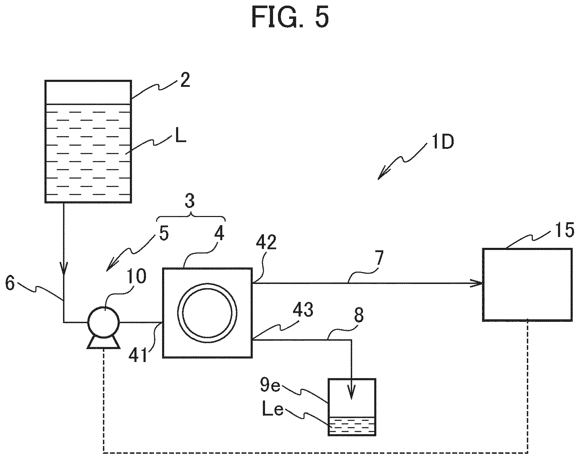

When only one of the segments is to be subjected to testing in the microorganism screening system 1C in FIG. 4, branching of the flow channels 7 and 8 is not required. Therefore, for example, as in the microorganism screening system 1D in FIG. 5, the flow channel 7 may be connected to one of the test device 15 and the recovery container 9e, and the flow channel 8 may be connected to the other one. In FIG. 5, the outlet port 42 and the test device 15 are connected through intermediation of the flow channel 7, and the flow channel 8 through which the second segment is discharged is connected to the recovery container 9e. With this, the first segment is subjected to testing by the test device 15. The connection may be established the other way around.

In the microorganism screening system 1D in FIG. 5, the liquid feeding unit 5 includes the flow rate control mechanism that controls the flow rate of the liquid supplied to the hydrodynamic separation device 4. Specifically, the test device 15 of the microorganism screening system 1D is electrically connected to the pump 10, and a signal for controlling a drive of the pump 10 can be transmitted to the pump 10. In other words, a drive of the pump can be electrically controlled based on information relating to the particle diameter measured by the test device 15. With this, the flow rate of the liquid supplied to the hydrodynamic separation device 4 can be adjusted. Therefore, the flow rate of the liquid L is changed through feedback of the measurement result, and the separation condition can be optimized so as to obtain the first segment containing the microorganism particles having a desired particle diameter.

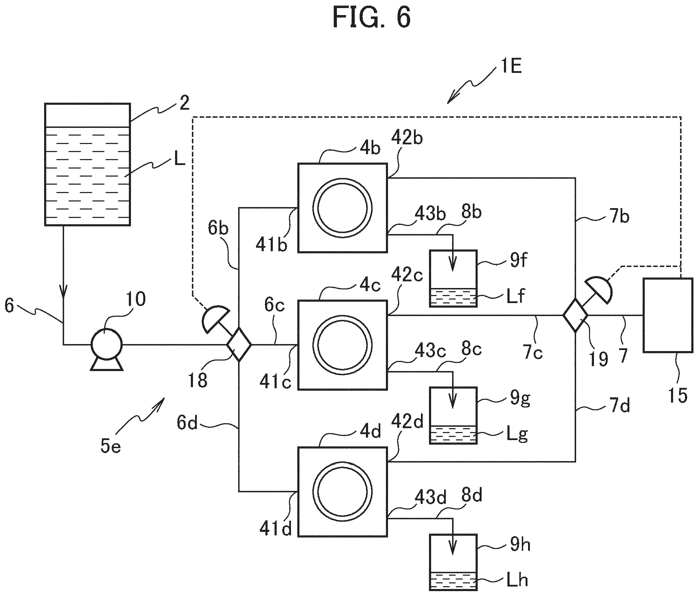

In the microorganism screening system in FIG. 5, the separation condition is optimized by adjusting the flow rate of the liquid supplied to the hydrodynamic separation device. In comparison, in a case where a plurality of hydrodynamic separation devices including curved flow channels with different designs are used, there may be adopted a configuration of changing the separation condition without changing the flow rate of the liquid. A microorganism screening system 1E in FIG. 6 corresponds to an embodiment obtained by modifying the system configuration in FIG. 5, and further includes at least one (two in FIG. 6) additional hydrodynamic separation device. In other words, the microorganism screening system 1E includes a plurality of (three in FIG. 6) hydrodynamic separation devices 4b, 4c, and 4d. A liquid feeding unit 5e includes a supply switching mechanism that is configured to supply the liquid L to one of those hydrodynamic separation devices, and at the same time, change a supply destination of the liquid L.

Specifically, in the microorganism screening system IE, the supply channel 6 that connects the container 2 and the hydrodynamic separation devices 4b, 4c, and 4d to each other is branched at the terminal end to have three flow channels 6b, 6c, and 6d that are connected to inlet ports 41b, 41c, and 41d of the hydrodynamic separation devices 4b, 4c, and 4d, respectively. In other words, the hydrodynamic separation devices 4b, 4c, and 4d are arranged in parallel with respect to the container 2. A switching valve 18 is provided at the branching point of the supply channel 6, the liquid L is supplied to one of the hydrodynamic separation devices 4b, 4c, and 4d, and the supply destination can be changed by the switching valve 18. The hydrodynamic separation devices 4b, 4c, and 4d include curved flow channels whose curvatures or rectangular cross-sectional shapes are different from one another. In other words, the separation conditions for hydrodynamic separation are different from one another. Thus, when the liquid is supplied at the same flow rate, the particles are separated in accordance with different particle diameters.

Outlet ports 42b, 42c, and 42d of the hydrodynamic separation devices 4b, 4c, and 4d are connected to flow channels 7b, 7c, and 7d, respectively. The terminal ends of those flow channels is integrated as one to the flow channel 7 via a switching valve 19, and are connected to the test device 15. The first segment separated from the liquid L supplied to one of the hydrodynamic separation devices 4b, 4c, and 4d is supplied to the test device 15. When connection of the switching valves 18 and 19 is adjusted to change the supply destination, those switching operations are inter-related. The test device 15 measures a particle diameter in the first segment. Outlet port 43b, 43c, and 43d of the hydrodynamic separation devices 4b, 4c, and 4d are connected to the recovery containers 9f, 9g, and 9h through intermediation of flow channels 8b, 8c, and 8d, respectively. Liquids Lf, Lg, and Lh separated as second segments in the hydrodynamic separation device are stored in recovery containers 9f, 9g, and 9h, respectively.

The test device 15 of the microorganism screening system 1E is electrically connected to the switching valves 18 and 19, and a signal for controlling connection switching of the switching valves 18 and 19 is transmitted based on the measurement result obtained by the test device 15. Therefore, the hydrodynamic separation device to be used can be selected and changed automatically under electric control so that the microorganism particles having a desired particle diameter can be obtained. In other words, the separation condition in this embodiment is optimized by changing hydrodynamic separation to be used in place of drive control of the pump 10.

The microorganism screening system 1E in FIG. 6 is configured so that the first segment is supplied from the hydrodynamic separation devices 4b, 4c, and 4d to the test device 15. In contrast, the configuration can be made so that the second segment is supplied to the test device 15. Further, the configuration of the flow channel connected to the outlet port of each of the hydrodynamic separation devices 4b, 4c, and 4d may be modified to that in the microorganism screening system 1C in FIG. 4. With this, any one of the first segment and the second segment that are separated from the liquid supplied to one of the hydrodynamic separation devices 4b, 4c, and 4d can be supplied to the test device. The measurement results on both the segments can be sequentially obtained by the test device through switching. In other words, by switching the supply destinations, the test device can perform measurement on at least one of the first segment and the second segment.

The microorganism screening system 1E in FIG. 6 can utilize the liquid L so that the microorganism particles are isolated into four segments in accordance with a particle diameter. For example, first, the liquid L in the container 2 is supplied to a hydrodynamic separation device selected from the hydrodynamic separation devices 4b, 4c, and 4d, which performs separation for the first segment having the smallest particle diameter. Then, the first segment recovered from the test device returns to the container 2. Subsequently, the liquid in the container 2 is supplied to another hydrodynamic separation device that performs separation for the medium particle diameter. Similarly, the first segment is recovered from the test device 15, and returns to the container 2. Finally, the same operation for supplying the liquid to the other hydrodynamic separation device that performs separation for the largest particle diameter is operated. The operations are repeated in this manner. With this, the particle diameter of the microorganism particles contained in the liquid that is sequentially recovered in each of the recovery container 9f, 9g, and 9h, and the test device 15 is increased in a stepwise manner. In this case, a circulation channel and a pump that cause the liquid to circulate from the test device 15 to the container 2 are provided. With this, the repeated operations can be performed automatically and continuously. This is advantageous in view of contamination prevention.

In the above-mentioned operations, a size of the particle contained in the isolated liquid can be confirmed through use of the test device 15. Thus, based on the measurement results, separation in one or two of the hydrodynamic separation devices 4b, 4c, and 4d may be omitted. Further, when change or adjustment of the separation condition is not required in the hydrodynamic separation device, particle diameter measurement may be omitted. Thus, the test device 15 may be omitted from the microorganism screening system 1E. In the description given above, the operations are repeated for the first segment. However, those repeated operations may be performed for the second segment. In this case, the flow channel configuration of the microorganism screening system 1E may be changed so that the second segment is supplied to the test device 15. First, the liquid L is supplied to the hydrodynamic separation device that performs separation for the second segment having the largest particle diameter.

The microorganism screening system 1E in FIG. 6 may further be modified so as to perform drive control of the pump, similarly to the microorganism screening system 1D in FIG. 5. In this case, the repeated operations described above are regarded as one unit, and a supply flow rate of the liquid is changed. With this, supply and hydrodynamic separation of the liquid can be performed. With this, the possible number of separation steps is a multiple of the number of hydrodynamic control devices included in the microorganism screening system. The microorganism screening system is designed based on a proper processing flow rate (rated flow rate) in the hydrodynamic separation device. A supply flow rate of the liquid may be controlled within the range that achieves a proper processing flow rate in the hydrodynamic separation device.

In addition to the above-mentioned microorganism size measurement performed by the test device 15, examples of measurement items for evaluating whether the target screening is performed include a shape of microorganisms, life/death determination, a cell cycle state, activity, a genetic test, and an analysis on a matter produced by microorganisms. Activity includes a proliferation function, metabolism activity, enzyme activity, and antibacterial activity. Therefore, the test device 15 may have functions capable of measuring one or more of the above-mentioned items, as required. Specifically, the above-mentioned items may be measured by performing operations such as culture, observation with a microscope, staining or light emission with a reagent, fluorescence detection, an electrophoresis, and absorption spectrum measurement through use of an electromagnetic wave such as an infrared spectrum. Further, the above-mentioned items may also be measured by utilizing an indicator such as a marker selectively reacting to a specific component, structure, gene, or the like and conducting an optical analysis through flow cytometry. Examples of the devices capable of performing those operations include a flow cytometer (product name: Attune NxT) available from Thermo Fisher Scientific Inc., a cell analyzer (product name: Vi-Cell) available from Beckman Coulter Inc., and a plate reader (product name: ViewLux) available from PerkinElmer, Inc.

Note that the second segment isolated through hydrodynamic separation contains relatively small microorganism cells, debris, and the like. A filter may be used to remove debris or aggregates from the liquid, and components contained therein may be analyzed. Further, refinement processing may be performed to recover a specific component. A filter may be selected with an appropriate hole diameter according to an object to be removed. Examples of the filter include a precision filtration film and an ultra-filtration film.

The microorganism screening system described above is used, thereby performing a screening method for various microorganisms. Separation in accordance with a microorganism particle size is performed, and thus screening for superior microorganism strains can be achieved, which assists efficient industrial application of fermentation and photosynthesis of microorganisms. Examples of the microorganism includes a procaryote (a eubacterium and an archaebacterium), a eucaryote (an alga, a protist, a fungus, and a myxomycete), and a virus. Specifically, examples of the procaryote include bacteria such as a colon bacillus, a hay bacillus, and Staphylococcus aureus, and hay bacilli such as actinomyces, cyanobacteria, and methanogenic bacteria. Examples of the eucaryote include algae such as spirogyra and wakame seaweed, protists such as a paramecium, and eumycetes such as mold, a yeast, a mushroom. Further, examples of the protists include amebas. Trochelminths such as a rotifer and microalgae such as Botryococcus braunii are also included as microorganisms.

Yeast and microalgae have been reported to have synthesis ability of oils and fats. Further, some photosynthetic bacteria, yeast, and lactic bacteria synthesize vitamins or biologically active substances. Actinomyces that generates various antibiotics are also known. Various useful substances such as oils and fats, proteins, vitamins, and enzymes produced by microorganisms are recovered, and may utilized in manufacturing various products such as pharmaceutical products, food and drink, and fuels. Hitherto, various fermentative microorganisms have been used in manufacturing processing for food and drink. For application of such microorganisms, screening for microorganism strains having specific characteristics or excellent novel functions can be performed. The microorganism strains excellent in a proliferation function, a metabolizing function, productivity, or the like contribute to improvement in productivity of useful components. Some microorganisms are mutated in such a way that useful components such as proteins stored inside a cell are released to the outside. In general, when screening for such microorganism strains is performed, potential microorganisms suitable for industrial application can be obtained. Further, even in a case of a plant being a multicellular organism, screening for desired cell particles can be performed for a single cell or a particle-like aggregate of a plurality of cells by applying the technique of the present disclosure.

A liquid subjected to hydrodynamic separation is a liquid containing microorganism particles and a liquid medium. As described above, the microorganisms cultured in the liquid culture medium may be directly used. The liquid culture medium may be any liquid culture medium such as a synthetic medium, a semi-synthetic medium, and a natural medium, and may contain a nutrient, a differentiating agent for the purpose of testing (such as a pH indicator, an enzyme substrate, and a sugar), a selective agent for suppressing growth of microorganisms other than target microorganisms, and the like. A liquid culture medium having low viscosity is preferably used so that hydrodynamic separation efficiently progresses. In a case of a microorganism particle such as a virus having a small particle diameter, a liquid culture medium to which a flocculation agent is added may be used to form a particle having a size that facilitates hydrodynamic separation. Examples of the flocculation agent include a polymer flocculation agent and an inorganic flocculation agent such as polyaluminum chloride, aluminum sulfate, and polysilica iron.

Even when microorganism particles contained in a liquid is under a state obtained by mixing a plurality types of microorganisms, screening for microorganism strains in accordance with a particle diameter can be performed. However, the liquid preferably contains a single species of microorganisms so that screening for target microorganism strains efficiently progresses. The microorganism particles of a single species are subjected to separation in the hydrodynamic separation device. With this, screening for target microorganism strains, which involves isolation of relatively large microorganism particles, is facilitated.

It is practical that the test device 15 of the microorganism screening system includes means for measuring at least one of the number of particles, particle diameter distribution, a survival rate of the microorganism particles, in addition to a particle diameter of the microorganism particles. Before the test device 15 conducts a test for the liquid obtained through hydrodynamic separation, the microorganisms may be cultured and increased. The microorganisms may be cultured in accordance with a usual method under culture conditions that have been known to be suitable for the microorganisms. The liquid culture medium used for culturing the microorganisms may be any liquid culture medium such as a synthetic medium, a semi-synthetic medium, and a natural medium, and a medium suitable for the microorganisms to be cultured may be selected and used as appropriate. In general, a selective enrichment medium or a selective separation medium prescribed for increasing a specific strain type is suitably used. The liquid culture medium may be selected and used from commercially available liquid culture media as appropriate, or may be produced in accordance with a known prescription through use of a nutrient, purified water, and the like. A differentiating agent for the purpose of testing (such as a pH indicator, an enzyme substrate, and a sugar), a selective agent for suppressing growth of microorganisms other than target microorganisms, and the like may be added as required. When the liquid medium being the liquid L subjected to hydrodynamic separation is a liquid culture medium used for culturing microorganisms, the microorganisms can be easily cultured.

When useful substances produced by the microorganisms are contained in the liquid, the segment on the inner circumferential side, which is recovered from the hydrodynamic separation device, is subjected to refinement. With this, the useful substances can be recovered. When produced useful substances are present inside cells of the microorganisms, the useful substances released from dead cells may be contained in the segment on the inner circumferential side, which is recovered from the hydrodynamic separation device. Thus, similarly, the useful substances can be recovered from the recovered segment.

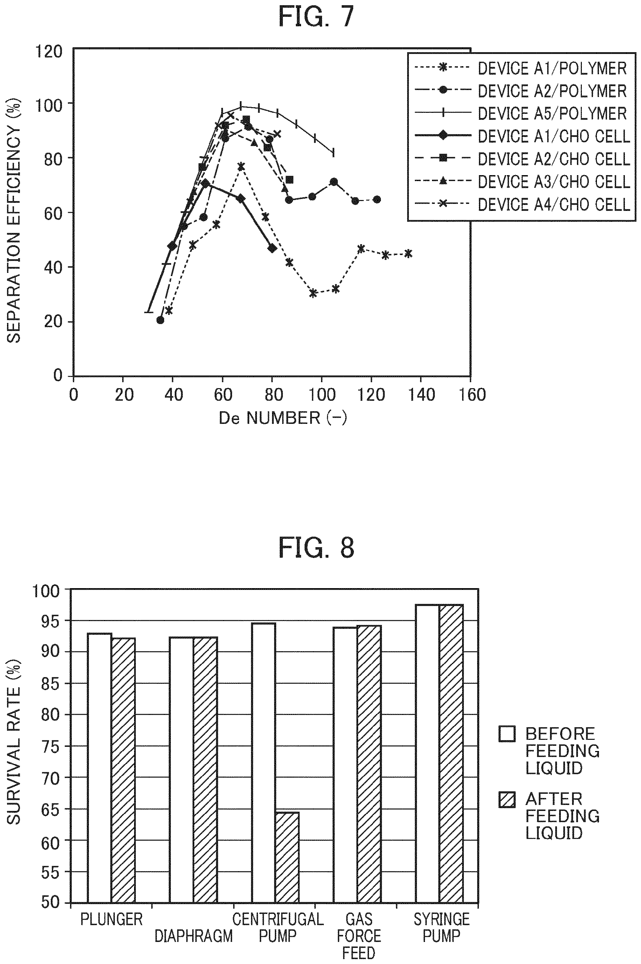

FIG. 7 is a graph showing results of examination on a relationship between separation efficiency and a De number in the hydrodynamic separation device. Specifically, any one of five types of hydrodynamic separation devices (devices A1 to A5) having different flow channel dimensions and any one of separation targets being polymer particles (styrene-divinylbenzene polymer particles) and Chinese hamster ovary cells (CHO cells) were used, and separation was performed in the hydrodynamic separation devices. Any one of the separation targets had a particle diameter average falling within a range from 14 µm to 18 µm. Separation efficiency is a value obtained by calculating [1-(x/X)]×100 (%), where, in the expression, X denotes a concentration of the separation target contained in the liquid before separation, and x denotes a concentration of the separation target contained in the segment on the inner circumferential side after separation. Any one of the separation targets had narrow particle diameter distribution. Thus, evaluation was given by regarding separation efficiency as 100% under a state in which the total amount of particles or cells concentrated in the segment on the outer circumferential side. As understood from the graph, separation efficiency was the highest with the De number around 70 in both the cases of the polymer particles and the animal cells. In general, high separation efficiency can be achieved under a condition where the De number falls within a range from 50 to 80. Therefore, it can be understood that, due to hydrodynamic separation, separation in accordance with a particle size can be performed regardless of a particle composition.

FIG. 8 shows results of examination on how a pump type, which fed a liquid culture medium to a hydrodynamic separation device, affected a survival rate of animal cells. The liquid culture medium for culturing cells was supplied to the hydrodynamic separation device at an ejection pressure of 0.3 MPa through use of a pump. Two segments containing liquid culture media discharged from the separation device were collectively gathered, and a small amount thereof was taken as samples. The results were obtained by measuring a survival rate of cells in each sample (%, a rate of living cells in the total cell numbers) through use of a cell analyzer (available from Beckman Coulter Inc., product name: Vi-Cell). "Gas force feed" in the graph indicates a mode in which the liquid culture medium was pressurized and fed by a compressed air from a pressure tank to which a compressor was attached, and can be categorized as a positive displacement pump. From FIG. 8, it can be understood that the cells were heavily damaged by a centrifugal pump and that reduction in survival rate was prevented with the other pumps categorized as a positive displacement pump. The microorganism particles had higher durability than the animal cells, and hence were not significantly affected by the pump types as in FIG. 8. Nevertheless, in a case where durability of microorganisms subjected to screening is unclear, a pump may be selected as appropriate.

As described above, the hydrodynamic separation technique is utilized, and thus the relatively large microorganism particles can be selectively concentrated and separated from the microorganism particles contained in the liquid. Thus, the microorganism strains having a proliferation function can be isolated. Hydrodynamic separation can promote concentration and separation for relatively large cells much more efficiently than centrifugal separation, and can perform concentration and separation with a higher survival rate without damaging microorganisms as compared to filter separation and the like. Desired microorganism strains may be recovered from the segment containing the relatively small microorganism particles after separation and removal. Further, a filter or the like may be used to remove unnecessary objects such as cell pieces and perform refinement. With this, components can be recovered. During hydrodynamic separation, the liquid is supplied to the curved flow channel at a relatively high speed. Thus, the microorganism screening system according to the present disclosure has high processing capacity of performing concentration and separation for cells. Efficiency in performing screening in accordance with a microorganism particle diameter is improved, and desired screening for microorganism strains can be performed practically. Thus, the present disclosure is sufficiently applicable to industrial use.

### Examples

With reference to a particle separator described in Patent Literature 1 given above, a resin molded body having a flat plate-like shape was produced. Inside the molded body, an arc-shaped curved flow channel (a turning diameter: approximately 115 mm, a radial cross-section of a flow channel: a rectangular shape, a flow channel width: approximately 3 mm) was formed. The molded body was used as a flow channel unit, and a hydrodynamic separation device was configured. A syringe pump (that pressurized and fed the liquid by pushing and pulling a plunger repeatedly) was used as the pump 10 for supplying the liquid, and the hydrodynamic separation device described above was used. In this manner, the microorganism screening system 1 in FIG. 1 was configured.

Yeast were added and dispersed in water. A yeast aqueous liquid containing yeast particles was thus produced, and was stored in the container 2 of the microorganism screening system 1. Through use of the syringe pump, the yeast aqueous liquid in the container 2 was pressurized and fed to the curved flow channel of the hydrodynamic separation device at a constant flow rate of 110 mL/min. The first segment on the outer circumferential side (containing relatively large yeast particles) and the second segment on the inner circumferential side (containing relatively small yeast particles), which were discharged from the outlet ports 42 and 43 of the hydrodynamic separation device, were recovered in the recovery containers 9a and 9b, respectively. For each of the segments, the number of yeast contained in the liquid and a survival rate (a rate of living yeast in the total number of yeast) were measured through use of an image type particle measurement device (available from Beckman Coulter Inc., product name: Vi-Cell). This operation and measurement were performed twice. The results obtained in each time are shown in Table 1.

**[Table 1]**

| | Average cell diameter [µm] | | | Survival rate of yeast [%] | | |
|---|---|---|---|---|---|---|
| | Yeast aqueous liquid | First segment | Second segment | Yeast aqueous liquid | First segment | Second segment |
| First time | 6.12 | 6.14 | 5.97 | 84.6 | 86.9 | 80.3 |
| Second time | 6.12 | 6.24 | 6.01 | 84.6 | 88.0 | 81.3 |

As understood from comparison regarding an average cell diameter in Table 1, it is clear that relatively small cells (cell diameter: around 6.0 µm) were contained in the second segment and that relatively large cells (cell diameter: around 6.2 µm) concentrated in the first segment. In other words, for both the first time and the second time, the yeast were separated in accordance with a cell diameter. Significant reduction of a survival rate of the yeast was not confirmed before and after hydrodynamic separation. Thus, it is concluded that a separation operation caused little damage on the yeast. Further, in the first segment, a survival rate of the yeast was significantly increased as compared to the yeast aqueous liquid before separation. The survival rate was increased because a rate of the relatively small particles contained in the first segment was reduced, and thus a rate of dead fungus bodies, debris, and impurities was reduced. Therefore, it is clear that hydrodynamic separation enabled the yeast having a relatively large particle diameter to be recovered as the first segment, and that the yeast with a high survival rate were obtained.

### INDUSTRIAL APPLICABILITY

The relatively large particles among the microorganism particles are selectively concentrated and isolated. With this, screening for the microorganism strains can be performed. Based on a particle diameter, efficient screening can be performed for the microorganism strains excellent in a proliferation function, a metabolizing function, productivity, or the like. The microorganism strains obtained through screening are utilized in manufacturing various products to which a biotechnology is applied. With this, this utilization contributes to economic efficiency and quality improvement when bio-related products are provided. Further, availability of products that are currently rear or expensive can be promoted.

## Claims

1. A microorganism screening system, comprising:
a container configured to store a liquid containing microorganism particles and a liquid medium; and
a microorganism separation device, wherein
the microorganism separation device includes:
a hydrodynamic separation device including a curved flow channel having a rectangular cross-section, and being configured to separate the liquid into a first segment containing relatively large microorganism particles and a second segment containing relatively small microorganism particles through use of a vortex flow generated in the liquid flowing through the curved flow channel; and
a liquid feeding unit configured to supply the liquid from the container to the hydrodynamic separation device.

2. The microorganism screening system according to claim 1, wherein
the hydrodynamic separation device includes:
a single inlet port through which the liquid is takes in; and
two outlet ports through which the first segment and the second segment are separately discharged, and
the relatively large microorganism particles are concentrated and are contained in the first segment.

3. The microorganism screening system according to claim 1 or 2, wherein
the liquid feeding unit includes:
a pipe configured to connect the container and the hydrodynamic separation device to each other; and
a pump configured to apply a hydrodynamic pressure to the liquid for supplying the liquid to the hydrodynamic separation device.

4. The microorganism screening system according to any one of claims 1 or 3, further comprising:
a test device configured to measure a size of a microorganism particle contained in the liquid, wherein
a flow rate of the liquid supplied from the liquid feeding unit to the hydrodynamic separation device is changed based on a measurement result on at least one of the first segment and the second segment, the measurement result being obtained by the test device.

5. The microorganism screening system according to claim 4, wherein
the liquid feeding unit includes a flow rate control mechanism configured to control a flow rate of the liquid supplied to the hydrodynamic separation device.

6. The microorganism screening system according to any one of claims 1 or 3, further comprising:
at least one additional hydrodynamic separation device including a curved flow channel with a curvature or a rectangular cross-sectional shape being different from that of the hydrodynamic separation device, wherein
the liquid feeding unit includes a supply switching mechanism configured to supply the liquid to one of the hydrodynamic separation device and the additional hydrodynamic separation device and, at the same time, change a supply destination.

7. The microorganism screening system according to claim 6, further comprising:
a test device configured to measure a size of a microorganism particle contained in the liquid, wherein
the supply switching mechanism is configured to change a supply destination of the liquid based on a measurement result on at least one of a first segment and a second segment that are obtained by separating the liquid supplied to one of the hydrodynamic separation device and the additional hydrodynamic separation device, the measurement result being obtained by the test device.

8. The microorganism screening system according to any one of claims 1 to 7, wherein
the microorganism particles include any one of a virus, a eubacterium, an archaebacterium, a fungus, a myxomycete, an alga, and a protist.

9. The microorganism screening system according to any one of claims 1 to 8, wherein
the microorganism particles are a single species, and screening for target microorganism strains is performed through separation in the hydrodynamic separation device.

10. The microorganism screening system according to any one of claims 4, 5, and 7, wherein
the test device further includes means for measuring at least one of the number of particles, particle diameter distribution, and a survival rate of microorganism particles.

11. A microorganism screening method of performing screening for microorganism particles in accordance with a particle size through use of a liquid containing the microorganism particles and a liquid medium, the microorganism screening method comprising:
a hydrodynamic separation step of supplying the liquid to a curved flow channel having a rectangular cross-section and separating the liquid into a first segment containing relatively large microorganism particles and a second segment containing relatively small microorganism particles through use of a vortex flow generated in the liquid flowing through the curved flow channel; and
an isolation step of isolating at least one of the first segment and the second segment.
